# EUROPEAN PATENT APPLICATION

(11) **EP 4 696 258 A1**
(43) Date of publication of application: **18.02.2026**
(21) Application number: 25195706.4
(22) Date of filing: 13.08.2025
(51) Int. Cl.: A61B 17/70, A61B 17/02

(54) **MINIMALLY INVASIVE SURGICAL SCREW DEVICE**

(30) Priority: 13.08.2024 TW 113130345
(71) Applicant: CHANPIN MEDTAK CO., LTD., New Taipei City 235 (TW)
(72) Inventor: TSUANG, Fon-Yih, 235 New Taipei City (TW); YANG, Chang-Che, 235 New Taipei City (TW); CHIANG, Yueh-Feng, 235 New Taipei City (TW); KUAN, Che-Yung, 235 New Taipei City (TW)
(74) Representative: Manitz Finsterwald Patent- und Rechtsanwaltspartnerschaft mbB

(57) **Abstract**

A MIS screw device is provided. The MIS screw device includes: a screw element; a positioning element having a limiting portion for receiving the screw element and an expanding portion connected to the limiting portion; and a flexible extension wing having two opposing first sections respectively coupled to opposite positions of the positioning element, wherein the two first sections extend in a direction parallel to a longitudinal direction of the positioning element. Thereby, by using the MIS screw device of the present disclosure, pedicle screws implantation can be performed while minimizing the wound area at the surgical site and reducing the space occupied by the surgical path from the skin to the subcutaneous tissue.

## Description

### CROSS-REFERENCE TO RELATED APPLICATIONS

This application claims the benefit of TW Patent Application No. 113130345, filed on August 13, 2024 in Taiwan, the entire content of which is incorporated herein by reference.

### BACKGROUND

### 1. Technical Field

The present disclosure relates to a minimally invasive surgical (MIS) device, and more particularly, to a MIS screw device for spinal surgery.

### 2. Description of Related Art

The spine is one of the most important structures responsible for maintaining mobility in the human body. When serious spinal lesions occurred (such as symptoms of spinal nerve compression), patients often unable to alleviate symptoms through correction and must rely on lumbar fusion surgery. A critical factor for the success of treatment lies in how to effectively stabilize and reposition vertebral body to prevent re-displacement.

At present, the most stable and commonly used treatment method is to fix the specific vertebrae to prevent displacement of the spinal segment. Each pedicle screw generally includes a screw shaft, a tulip part, and further a locking nut. In the surgical procedure, the pedicle screws are implanted in pairs into the vertebral from both sides of the spinous process at the pedicle region. A connecting rod is then bent to an appropriate curvature. The locking nuts are then tightened using surgical tools to secure the connecting rod into the tulip, thereby completing the repositioning or stabilization of the upper and lower vertebral bodies.

However, conventional spine surgery is performed as open surgery. A midline incision is made on the patient's back. The muscle tissue is dissected and retracted to the lateral sides of the vertebrae. The periosteum is then stripped to expose the vertebrae, after which the aforementioned pedicle screws are implanted. The surgical approach not only results in the large wound and significant blood loss, but also leads to delayed wound healing due to the destruction of anatomical structures.

Therefore, in recent years, minimally invasive spinal(MIS) surgery which can reduce wound size, has increasingly become the preferred choice for both surgeons and patients, and its clinical significance growing steadily. Generally, MIS is defined by a smaller and paramedial incision that aims to minimize damage to soft tissue. In MIS, extension components of tulip or sleeves are commonly used to maintain the surgical path. Specifically, the pedicle screws equipped with extension components or sleeves for implantation into the vertebral body. The extension components or sleeves are used to preserve the surgical path, allowing surgical tools and accessories to be operated on the pedicle screws, and enabling proper installation on a sufficient surgical field is established or unobstructed access is ensured.

However, it is known that the extension components of conventional pedicle screws are cylindrical or partially cylindrical in shape, so the surgical incision for instrumentation must be large enough in order to prevent interference between the extension parts of adj acent pedicle screws. Consequently, the surgical incisions inevitably requires a certain width (e.g., at least 5 cm), making it difficult to achieve the desired minimization of wound size.

Therefore, there is an urgent need in the art for minimally invasive screw devices that can overcome the above problems.

### SUMMARY

The present disclosure provides a minimally invasive surgical(MIS) screw device, including: a screw element; a positioning element having a first side and a second side opposite the first side, wherein the positioning element includes a limiting portion disposed on the first side for receiving the screw element, and an expanding portion connected to the limiting portion and extending toward the second side; and a flexible extension wing having two opposing first sections respectively coupled to opposite positions of the positioning element, and wherein two first sections extend in a direction parallel to a longitudinal direction of the positioning element.

In at least one embodiment of the aforementioned MIS screw device, the limiting portion includes a recessed opening through which the screw element is inserted into the limiting portion in a relatively rotatable manner.

In at least one embodiment of the aforementioned MIS screw device, the expanding portion includes two first guide portions, and the two first sections of the flexible extension wing are extended through the two first guide portions to be secured to the positioning element. For example, in certain embodiments, the first guide potions are disposed of a peripheral surface of the expanding portion. Furthermore, in certain embodiments, the limiting portion includes two second guide potions, respectively corresponding to each of the first guide potions, and the two first sections extend through the first guide potions and further extended through the two second guide potions.

In at least one embodiment of the aforementioned MIS screw device, the flexible extension wing further includes two second sections respectively connected to each of the first sections. The first bending point is provided between each the first section and the corresponding second section. The first bending point is bent so that the first section and the second sections form an angle ranging from 60 to 120 degrees. For example, the first section and the second section of the flexible extension wing are at an angle of 90 degrees or an L-shaped.

In at least one embodiment of the aforementioned MIS screw device, the second section of the flexible extension wing includes at least a second bending point.

In at least one embodiment of the aforementioned MIS screw device, the limiting portion includes a first side wall and a second side wall opposite to the first side wall, wherein an accommodating space for receiving the screw element is defined by the first side wall, the second side wall and the recessed opening, and the inner surfaces of the first side wall and the second side wall are formed with positioning threads.

In at least one embodiment of the aforementioned MIS screw device, the expanding portion includes guide threads.

In at least one embodiment of the aforementioned MIS screw device, the expanding portion includes a first support wall and a second support wall opposite to the first support wall, wherein the first support wall and the second support wall are respectively connected to the limiting portion and extend toward the second side of the positioning element to define a guide channel that extends to the limiting portion.

In at least one embodiment of the aforementioned MIS screw device, a width of the first support wall and the second support wall adjacent to the second side of the positioning element is less than a width of the first support wall and the second support wall adjacent to the first side of the positioning element.

In at least one embodiment of the aforementioned MIS screw device, a width of the flexible extension wing is less than or equal to a width of the first support wall and the second support wall located on the second side of the positioning element.

In at least one embodiment of the aforementioned MIS screw device, the flexible extension wing is formed from biocompatible flexible materials including metal wires, or alloy wires, shape-memory alloys, polymeric fibers, or composites thereof.

As can be understood from the above, in the MIS screw device provided by the present disclosure, the positioning elements are used to retract muscle tissues and the flexible extension wing is used to retract the epidermis, dermis, and subcutaneous tissues, wherein the volume or width of the flexible extension wing is significantly smaller than the positioning element. Therefore, compared with the cylindrical extension component of conventional pedicle screw, the use of the MIS screw device of the present disclosure can perform the installation of the pedicle screw while minimizing the scope of the wound on the surgical site, and reduces the space occupied of the surgical path from the skin to the subcutaneous tissues.

### BRIEF DESCRIPTION OF THE DRAWINGS

FIG. 1 is a schematic perspective view of a minimally invasive screw device for spinal surgery according to one embodiment of the present disclosure.
FIG. 2 is a schematic side view of the minimally invasive screw device for spinal surgery according to one embodiment of the present disclosure.
FIG. 3 is a schematic side view illustrating the minimally invasive screw device for spinal surgery according to one embodiment of the present disclosure, in a bent state at a first bending point.
FIG. 4 is a schematic side view illustrating a screw element rotating relative to a positioning element according to one embodiment of the present disclosure.
FIG. 5 is a schematic side view illustrating two minimally invasive screw devices in a first usage aspect according to one embodiment of the present disclosure.
FIG. 6 is a schematic perspective side view illustrating the two minimally invasive screw devices of FIG. 5 in a first usage aspect being fixed to the vertebral body.
FIG. 7 is a schematic side view illustrating two minimally invasive screw devices in a second usage aspect according to one embodiment of the present disclosure.
FIG. 8 is a schematic side perspective view illustrating the two minimally invasive screw devices of FIG. 7 in a second usage aspect being fixed to the vertebral body.

### DETAILED DESCRIPTION

Implementations of the present disclosure are described below by specific embodiments. One of ordinary skill in the art can readily appreciate other advantages and technical effects of the present disclosure upon reading the content of this specification. However, specific embodiments provided are not intended to limit the scope of present disclosure. The present disclosure can also be implemented or applied by other different implementations, various details contained in the content of this specification in accordance with different aspects and applications can also be modified or altered in different ways without departing from the scope of the present disclosure.

It should be noted that the structures, ratios, sizes and the like shown in the drawings appended to this specification are provided in conjunction with the disclosure of this specification for those skilled in the art to read and understand, rather than to limit the implementable scope of the present disclosure, thus any modifications to the structures, changes to ratio relationships, or adjustments to sizes shall be construed as falling within the scope of the technical content disclosed herein, as long as they do not affect the objectives and technical effects achieved by the present disclosure.

The approximate language used herein may be used to modify any quantitative expression within an acceptable range, such that it does not result in a change to the basic functionality associated therewith. Accordingly, a value modified by one or more terms such as "about" or the like is not limited to the precise value specified. In some embodiments, this approximation language may correspond to the precision or tolerance of the instrument used to measure the value.

It should be noted that terms such as "comprise", "include", "have" or "with" used herein with specific elements or features are intended to be open-ended. That is, such terms do not exclude the presence of other elements, components, compositions, structures, regions, parts, portions, devices, systems, steps, connections or relationships and the like not expressly recited.

The terms "upper", "lower", "front" and "rear" used herein are only used to facilitate the explanation of specific embodiments of the present disclosure and are not intended to limit the scope of the present disclosure. Variations, substitutions, or changes in relative positions or spatial relationships of elements shall be construed as within the scope of the present disclosure, so long as they do not substantially alter the technical content thereof.

It should be noted that sequential terms such as "first" and "second" recited herein are merely for convenience in describing or distinguishing elements, components, group compounds, structures, regions, portions, parts, devices, systems, and the like components in convenience, rather than to limit the implementable scope of the present disclosure, nor to imply any spatial, numerical, or temporal order. Furthermore, unless otherwise specified, singular-form terms such as "one" and "the" herein may also encompass the plural-form, and the term "or" herein can be used interchangeably with the term "and/or."

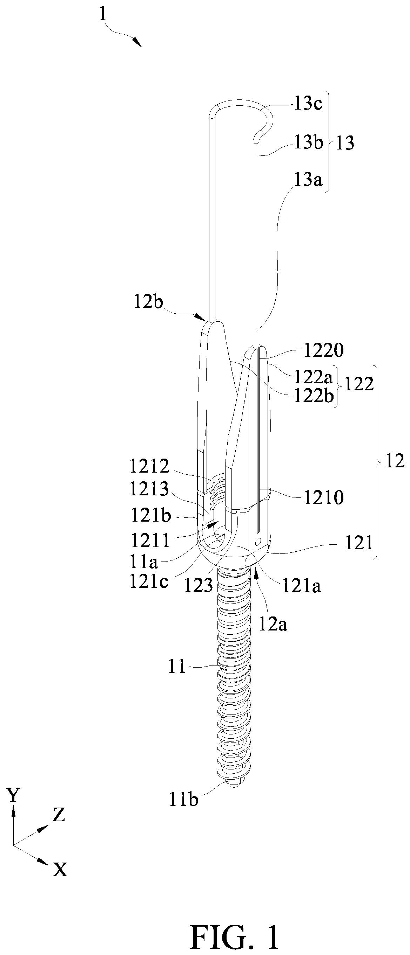

FIG. 1 is a schematic perspective view of a minimally invasive screw device 1 for spinal surgery according to one embodiment of the present disclosure. As shown in FIG. 1, a minimally invasive screw device 1 for spinal surgery includes a screw element 11, a positioning element 12 and a flexible extension wing 13. The positioning element 12 has a first side 12a and a second side 12b opposite to the first side 12a. A limiting portion 121 is disposed on the first side 12a and receives the screw element 11. An expanding portion 122 is connected to the limiting portion 121 and extends to the second side 12b. Furthermore, an incision 123 may be formed between the limiting portion 121 and the expanding portion 122.

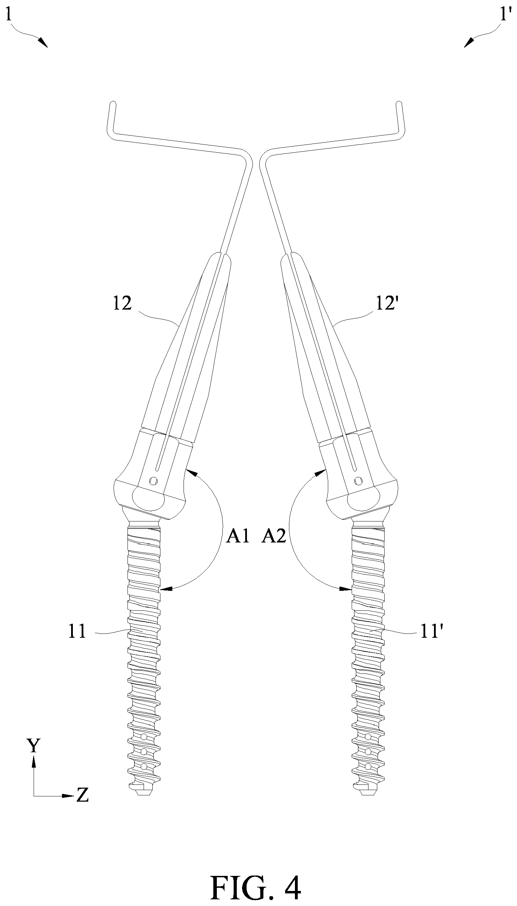

In at least one embodiment of the present disclosure, as shown in FIG. 1, a recessed opening 121c is formed at a bottom of the limiting portion 121 of the positioning element 12, so as to receive the screw element 11 within the limiting portion 121. More specifically, the screw element 11 has a first end 11a and a second end 11b. The first end 11a is received in the recessed opening 121c of the limiting portion 121, so that the screw element 11 extends through the recessed opening 121c and into the limiting portion 121 in a rotatable manner relative to the positioning element 12 (see FIG. 4, that is, the expanding portion 122 of the positioning element 12 can be oriented in different directions).

In at least one embodiment of the present disclosure, the screw element 11 is a metallic screw element, such as a pointed-end screw, with the first end 11a being the head portion and the second end 11b being the tip portion. Alternatively, the first end 11a of the screw element 11 may have a ball joint structure, so that the screw element 11 can rotate more smoothly after being coupled to the recessed opening 121c (i.e., a so-called universal screw structure or a multi-axial screw structure). It should be understood that the screw element 11 may be configured in various forms as needed and is not limited to the examples described above.

In at least one embodiment of the present disclosure, as shown in FIG. 1, the limiting portion 121 includes a first side wall 121a and a second side wall 121b opposite thereto. The first side wall 121a, the second side wall 121b and the recessed opening 121c together define an accommodating space 1211 for receiving the screw element 11.

In one embodiment, the front view or rear view (as the Z-axis direction of the coordinates shown in FIG. 1) of the limiting portion 121 shows two transverse openings 1213, which is a U-shape, communicated with the accommodating space 1211. That is, the first side wall 121a and the second side wall 121b are formed as two sides of the U-shape to form the transverse openings 1213, and the recessed opening 121c is formed as a bottom edge of the U-shape. Accordingly, a connecting rod (not shown) can be placed in the transverse openings 1213 and within a remaining portion of the accommodation space 1211 after receiving the screw element 11 in the limiting portion 121, thereby connecting the limiting positions 121 of two adjacent minimally invasive screw devices 1.

In at least one embodiment of the present disclosure, as shown in FIG. 1, inner surfaces of the first side wall 121a and the second side wall 121b of the limiting portion 121 are provided with positioning threads 1212. The minimally invasive screw device 1 further includes a fixing member (not shown), such as a locking nut. The fixing member is threaded along the positioning threads 1212 into the accommodating space 1211 and screwed therein to press the connecting rod into the limiting portion 121, that is, the connecting rod is positioned between the locking nut and the first end 11a of the screw element 11.

Furthermore, in this embodiment, the pressing force exerted by the locking nut to the connecting rod will also restricts the rotation between the screw element 11 and the positioning element 12 through the connecting rod. As a result, the angle between the screw element 11 and the positioning element 12 is fixed.

In at least one embodiment of the present disclosure, as shown in FIG. 1, the expanding portion 122 includes a first support wall 122a and a second support wall 122b opposite thereto. The first and second support walls 122a, 122b are respectively connected to the first and second side walls 121a, 121b of the limiting portion 121, and extend toward the second side 12b of the positioning element 12, thereby defining a guide channel that extends to the limiting portion 121 and communicates with the accommodating space 1211 thereof.

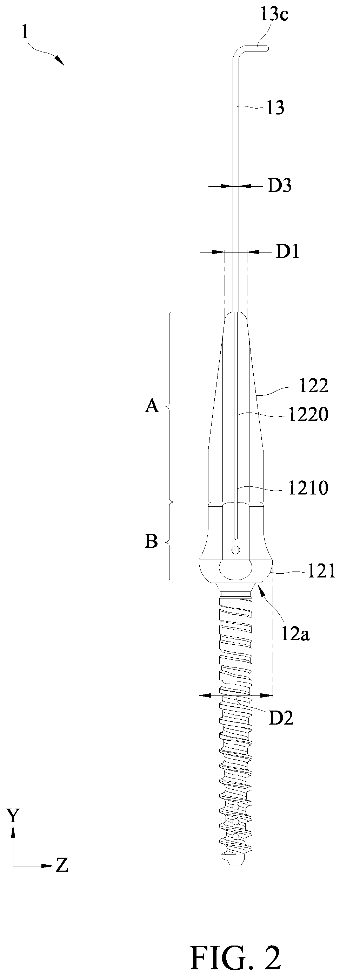

Furthermore, as shown in FIG. 2, the width D1 of the first and second support walls 122a, 122b adjacent to the second side 12b of the positioning element 12 is less than the width D2 adjacent to the first side 12a thereof.

In at least one embodiment of the present disclosure, a first guide portion 1220 is provided on the first support wall 122a and the second support wall 122b of the expanding portion 122. For example, the first guide portion 1220 is formed on the peripheral surface of each support wall 122a, 122b to define a chute-like structure, allowing the flexible extension wing 13 of the minimally invasive screw device 1 to pass through the first guide portions 1220 engage with the positioning element 12. It should be understood that the first guide portions 1220 formed on the first support wall 122a and the second support wall 122b may vary as needed and are not limited to the number shown in FIG. 1.

In some embodiments, the first guide portion 1220 is formed through the first and second support walls 122a, 122b of the expanding portion 122 to define a channel that extends along the Y-axis direction (as shown in the coordinates of FIG. 1) and penetrates both the first support wall 122a and the second support wall 122b.

Furthermore, in some embodiments, the inner surfaces of the first and second support walls 122a, 122b of the expanding portion 122 provided with guide threads (not shown) to facilitate the guidance of the fixing member (such as locking nuts) toward the accommodating space 1211. Specifically, the locking nut is threaded along the guide threads of the expanding portion 122 to the positioning threads 1212 of the limiting portion 121, where it presses the connecting rod into the limiting portion 121.

FIG. 2 is a side view of a minimally invasive screw device 1 for spinal surgery according to one embodiment of the present disclosure. As shown in FIG. 1 and FIG. 2, the first side wall 121a of the limiting portion 121 is provided with a second guide part 1210, and the second side wall 121b of the limiting portion 121 is also provided with a second guide part 1210 disposed opposite to the second guide part 1210 of the first side wall 121a (not visible in the view of FIG. 1 and FIG. 2 due the viewing angles).

In at least one embodiment of the present disclosure, as shown in FIG. 2, the second guide portions 1210 of the limiting portion 121 correspond to the first guide portions 1220 of the expanding portion 122 and may extend to the limiting portion. 121, such that the first guide portions 1220 are in communication with the second guide portions 1210.

In this embodiment, the flexible extension wing 13 of the minimally invasive screw device 1 has two opposite first sections 13a, which are respectively coupled to different positions of the positioning element 12, and the two first sections 13a are extended in a longitudinal direction of the positioning element 12. For example, the first sections 13a penetrate through the first guide portions 1220 of the extending parts 122 and are further penetrated the second guide portions 1210, thereby allowing the flexible extension wing 13 to be coupled to the limiting portion 121 of the positioning element 12.

In some embodiments, the flexible extension wing 13 of the minimally invasive screw device 1 is securely fixed on the positioning element 12. For example, the flexible extension wing 13 is disposed and fixed in the channel defined by the first guide part 1220 and the second guide part 1210 of the expanding portion 122. Furthermore, in some embodiments, the flexible extension wing 13 is integrally formed with the positioning element 12.

Furthermore, in one embodiment of the present disclosure, as shown in FIG. 2, the flexible extension wing 13 includeds a fixing portion 13c. The fixing part 13c is bent at, for example, 90 degrees with the other parts of the flexible extension wing 13. That is, the fixing part 13c of the flexible extension wing 13 extends along the Z-axis direction of the coordinates shown in FIG. 2, and except for the fixing part 13c, other parts of the flexible extension wing 13 extends along the Y-axis direction of the coordinates shown in FIG. 2.

In some embodiments, the flexible extension wing 13 coupled to the positioning element 12 forms a closed loop via the fixing part 13c and the positioning element 12. For example, the flexible extension wing 13 may be shape as an inverted U-shaped elongated structure, and can be fabricated from various biocompatible materials that are sufficiently rigid yet flexible, allowing the flexible extension wing 13 to still have a certain degree of support despite being deformable. For example, the flexible extension wing 13 may be formed from metal wires, alloy wires, shape-memory alloys, polymeric fibers, or composites thereof, and a width D3 of the flexible extension wing 13 is less than or equal to a width D1 of the first support wall 122a and the second support wall 122b at the second side 12b of the positioning element 12, as shown in FIG. 2. Furthermore, the space formed by the closed loop between the flexible extension wing 13 and the positioning element 12 facilitates positioning of the connecting rod.

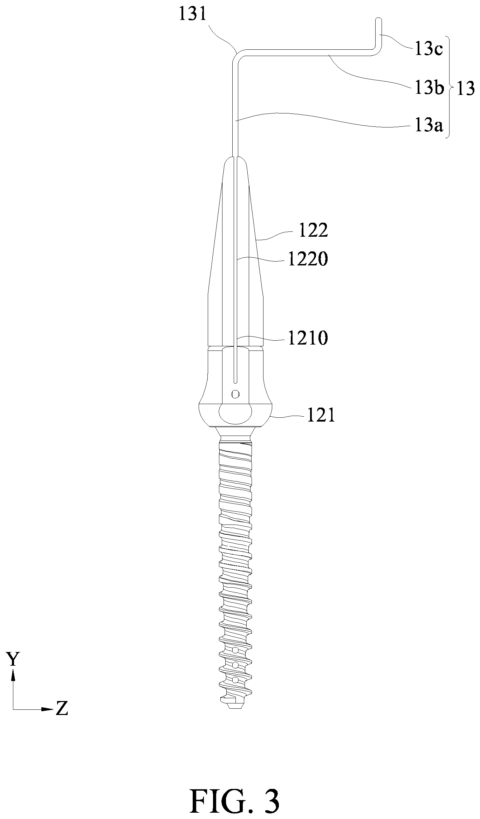

FIG. 3 is a schematic side view of a minimally invasive screw device 1 for spinal surgery according to one embodiment of the present disclosure, illustrating a state in which the device is bent at a first bending point. As shown in FIG. 3, the flexible extension wing 13 of the minimally invasive screw device 1 includes a first section 13a, a second section 13b connected to the first section 13a, and a fixing portion 13c connected to the second section 13b. A first bending point 131 is defined between the first section 13a and the second section 13b.

In some embodiments, the first bending point 131 of the flexible extension wing 13 may be selectively positioned according to user requirements, i.e., the user may arbitrarily determine the position at which the flexible extension wing 13 is to be bent.

In one embodiment of the present disclosure, as shown in FIG. 3, a portion of the first section 13a of the flexible extension wing 13 is disposed through the first guide part 1220 of the expanding portion 122 and the second guide part 1210 of the limiting portion 121, while a remaining portion of the first section 13a and the second section 13b are exposed outside the positioning element 12.

As shown in FIG. 3, the first sections 13a and the second sections 13b of the flexible extension wing 13 are bent at a 90 degrees angle at the first bending point 131 as the turning point. That is, the first sections 13a are extended along the Y-axis of the coordinate system shown in FIG. 3, and the second sections 13b are extended along the Z-axis of the coordinate system shown in FIG. 3, so that the first sections 13a and the second sections 13b of the flexible extension wing 13 are presented an L-shaped.

It should be understood that the angle formed between the first section 13a and the second section 13b bent at the first bending point 131 may vary as required (for example, a V-shape configuration having an angle less than 90 degrees shown in FIG. 4), but is not limited to the example described above.

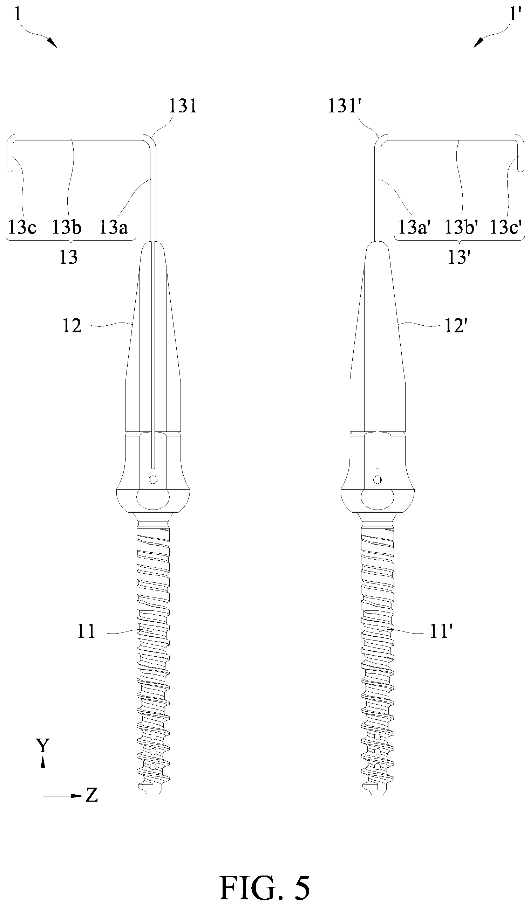

In some embodiments, the first sections 13a and the fixing part 13c of the flexible extension wing 13 are extended along the Y-axis of the coordinates system shown in FIG. 3, while the second sections 13b are extended along the Z-axis of the coordinates system shown in FIG. 3, whereby the flexible extension wing 13 are presented in a Z-shaped (as shown in the side view of FIG. 3) or a reversed U-shaped (as shown in the side view of FIG. 5).

Furthermore, in some embodiments, the second sections 13b of the flexible extension wing 13 include at least one second bending point (not shown), whereby in addition to the angle being formed between the first sections 13a and the second sections 13b, the second sections 13b themselves can also be bent through the second bending point to generate at least one bending angle. For example, through the first bending point 131 and the second bending point, the first sections 13a and the second sections 13b can form a reversed U-shape (excluding the fixing part 13c).

FIG. 4 is a schematic side view of a screw element 11, 11' rotating relative to a positioning element 12. 12' according to one embodiment of the present disclosure. As shown in FIG. 4, the positioning element 12 of the minimally invasive screw device 1 is angled rightward along the Z-axis direction to form an angle A1 with the screw element 11, while the positioning element 12' of the minimally invasive screw device 1' is angled leftward along the Z-axis direction to form an angle A2 with the screw element 11'. It should be understood that when the screw elements 11 and 11' are respectively fixed at specific positions, the angle A1 between the positioning element 12 and the screw element 11 and the angle A2 between the positioning element 12' and the screw element 11' can be freely adjusted to form a desired angle.

In one embodiment of the present disclosure, the rotation between the positioning element 12 and the screw element 11 is not limited to the plane of the Y-axis and the Z-axis. The rotation can also occur on the plane of the X-axis and the Y-axis or the plane of the X-axis and the Z-axis. In other words, the positioning element 12 and the screw element 11 are rotatable within a spatial range constrained by structural limits.

FIG. 5 is a schematic side view of two minimally invasive screw devices 1, 1' in a first usage mode according to one embodiment of the present disclosure. As shown in FIG. 5, the minimally invasive screw devices 1 and 1' of the present disclosure are presented in a first usage aspect. In the first usage mode, the flexible extension wing 13 of the minimally invasive screw device 1 is bent at the first bending point 131 to the left in the Z-axis. In other words, the second sections 13b of the flexible extension wing 13 are bent at the first bending point 131 about 90 degrees to the left in FIG. 5, so that the fixing part 13c of the flexible extension wing 13 originally located in the Z-axis direction, becomes almost parallel to the first section 13a.

Similarly, in the first usage mode, the flexible extension wing 13' of the minimally invasive screw device 1' is bent at the first bending point 131' to the right in the Z axis, that is, the second section 13b' of the flexible extension wing 13 ' is bent at the first bending point 131' approximately 90 degrees to the right in FIG. 5, so that the fixing part 13c' of the flexible extension wing 13' originally located in the Z-axis direction is almost parallel to the first section 13a'.

It can be seen from the above that the first usage mode of the present disclosure is to provide two minimally invasive screw devices 1, 1', and the flexible extension wing 13 of the minimally invasive screw device 1 and the flexible extension wings 13' of the minimally invasive screw device 1' are bent relative to each other while the fixing parts 13c and 13c' oriented downward to form a pair of extension wings.

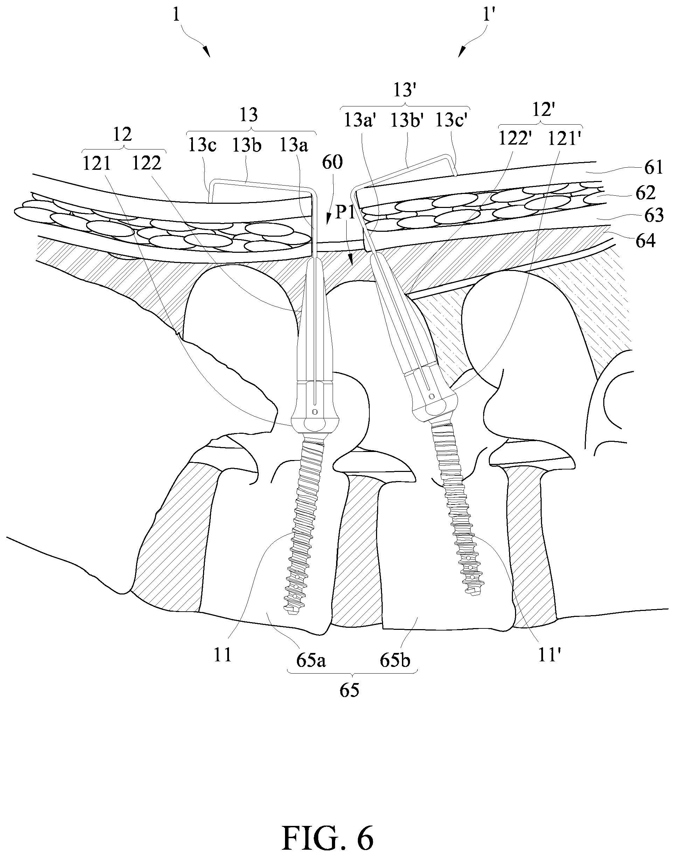

FIG. 6 is a schematic side perspective view of the two minimally invasive screw devices 1, 1' in a first usage aspect of FIG. 5 being fixed on a vertebral body 65. As shown in FIG. 6, when performing an operation of fixing the minimally invasive screw device on the vertebral body 65, an incision 60 is firstly made in the epidermal layer 61 corresponding to a position to be fixed (such as vertebras 65a, 65b shown in FIG. 6). The incision 60 then extends downward in sequence through a dermis layer 62, subcutaneous tissues 63 and muscle tissues 64 to form a surgical path P1, allowing the operator to view the vertebras 65a and 65b of the vertebral body 65.

Next, the minimally invasive screw device 1 and the minimally invasive screw device 1' are implanted into the vertebral body 65 from the opening 60 along the surgical path P1, and respectively screwed into the vertebra 65a and the vertebra 65b with the screw element 11 and the screw element 11' respectively. As shown in FIG. 6, the positioning element 12 of the minimally invasive screw device 1 is disposed on the vertebra 65a in a manner that allows rotation relative to the screw element 11. The expanding portion 122 of the positioning element 12 supports the muscle tissue 64 along the surgical path P1, and the epidermis layer 61, the dermis layer 62 and the subcutaneous tissues 63 are spread apart of the flexible extension wing 13 that is exposed from the first section 13a of the positioning element 12.

Similarly, the positioning element 12' of the minimally invasive screw device 1' is disposed on the vertebra 65b in a manner that allows rotatable relatition to the screw element 11'. The expanding portion 122' of the positioning element 12' supports the muscle tissue 64 along the surgical path P1, and the epidermis layer 61, the dermis layer 62 and the subcutaneous tissues 63 are spread apart of the flexible extension wings 13' exposed from the first section 13a' of the positioning element 12'. In view of the fact that the elasticity of the epidermis layer 61, the dermis layer 62 and the subcutaneous tissues 63 is relatively smaller than the elasticity of the muscle tissues 64, only a relatively small force is required to spread apart the epidermal layer 61, dermal layer 62, and subcutaneous tissue 63 to form the surgical path P1.

Therefore, the flexible extension wings 13, 13' can be implemented in the form of a metal wire structures, the dimensions or width of which is significantly smaller than the supporting wall structure of the expanding portions 122, 122'. The size of the opening 60 can be subsequently be adjusted by pulling and tensioning second sections 13b, 13b' of the flexible extension wings 13, 13' and be adjusted by setting the fixing parts 13c, 13c' on the epidermal layer 61. When the flexible tension wings are released from tension, the opening 60 returns to the original incision size. Accordingly, the size of the opening 60 does not need to correspond to the projection range of the vertebra 65a and the vertebra 65b, nor is it necessary to enlarge the opening 60 based on the number of implanted minimally invasive screw devices, thereby achieving a minimized surgical opening. According to the above description, since the expanding portions 122 and 122' of the positioning elements 12 and 12' are used to hold the muscle tissues, the length of the positioning elements 12 and 12' do not need to be excessively long and may simply range from 50 to 150 mm.

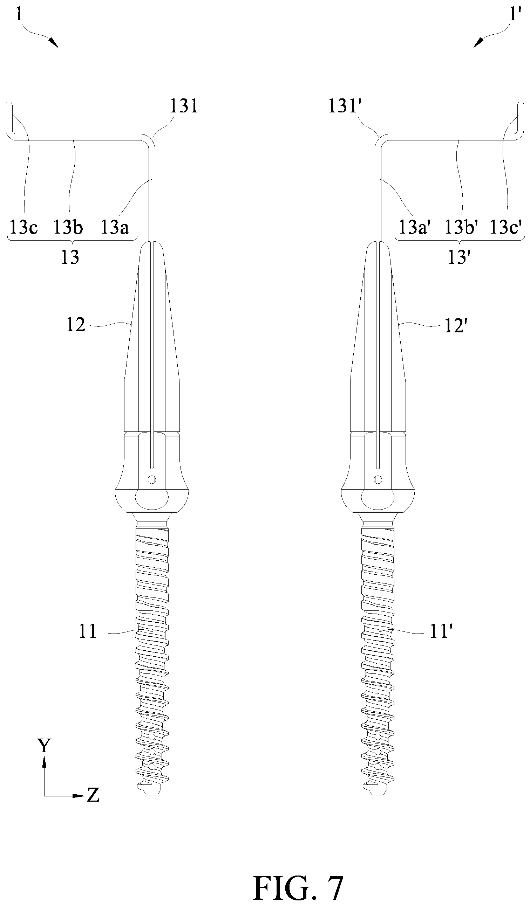

FIG. 7 is a schematic side view of two minimally invasive screw devices 1, 1' in a second usage mode according to one embodiment of the present disclosure. As shown in FIG. 7, the minimally invasive screw devices 1 and 1' of the present disclosure are presented in a second usage mode. In the second usage aspect, the flexible extension wing 13 of the minimally invasive screw device 1 is bent at the first bending point 131 to the left in the Z-axis. In other words, the second sections 13b of the flexible extension wing 13 are bent at the first bending point 131 about 90 degrees to the left in FIG. 7, so that the fixing part 13c of the flexible extension wing 13 originally located in the Z-axis direction is almost parallel to the first section 13a.

Similarly, in the second usage mode, the flexible extension wing 13' of the minimally invasive screw device 1' is bent at the first bending point 131' to the right in the Z axis, that is, the second section 13b' of the flexible extension wing 13 ' is bent at the first bending point 131' approximately 90 degrees to the right in FIG. 7, so that the fixing part 13c' of the flexible extension wing 13' originally located in the Z-axis direction is almost parallel to the first section 13a'.

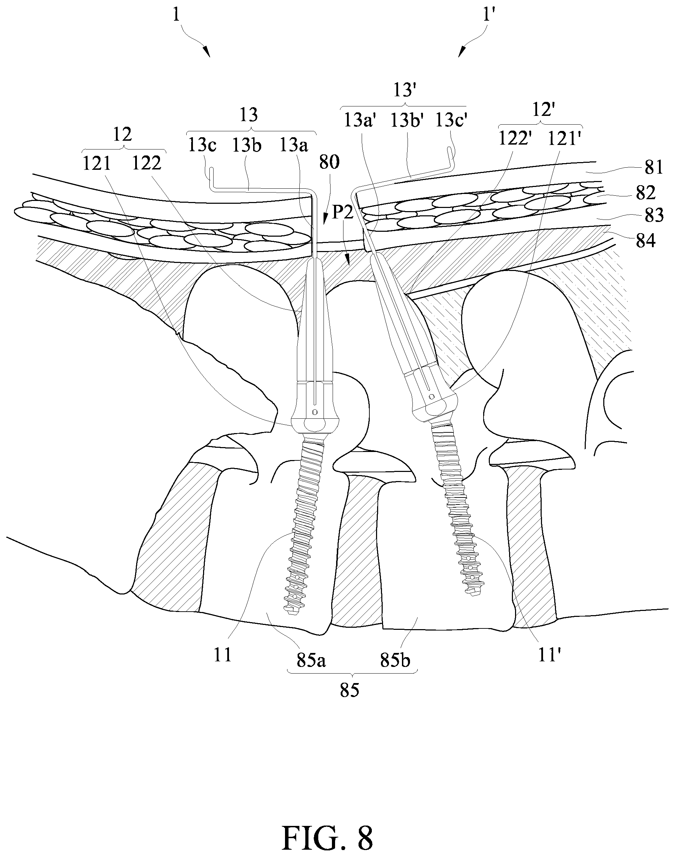

It can be seen from the above that the second usage mode of the present disclosure is similar to the above-mentioned first usage mode, in that two minimally invasive screw devices 1, 1' are provided, and the flexible extension wings 13 of the minimally invasive screw device 1 and the flexible extension wings 13' of the minimally invasive screw 1' are bent opposite to each other. However, the difference lies in that, in the second use mode, the fixing parts 13c and 13c' are directed upward, thereby forming a paired configuration of extending wings. Furthermore, the functional difference between the first usage mode and the second usage mode is that the main function of the first usage mode is to firmly fix the minimally invasive screw devices by pointing the fixing parts 13c and 13c' downward on the epidermal layer 61; and the main function of the second usage aspect is to adjust the size of the incision 80 by pointing the fixing parts 13c and 13c' upward, thereby achieving minimization of the opening during surgery (see FIG. 8).

FIG. 8 is a schematic side perspective view of the two minimally invasive screw devices 1, 1' in a second usage mode of FIG. 7 being fixed on a vertebral body 85. As shown in FIG. 8, when performing an operation of fixing the minimally invasive screw device on the vertebral body 85, an incision 80 is first created in the epidermal layer 81 corresponding to a position to be fixed (such as vertebras 85a, 85b shown in FIG. 8). The incision 80 extends downward sequentially through a dermis layer 82, subcutaneous tissues 83 and muscle tissues 84 sequentially to form a surgical path P2, allowing the operator to view the vertebras 85a and 85b of the vertebral body 85.

Next, the minimally invasive screw device 1 and the minimally invasive screw device 1' are implanted into the vertebral body 85 from the surgical incision 80 along the surgical path P2, and are screwed to the vertebra 85a and the vertebra 85b with the screw element 11 and the screw element 11' respectively. As shown in FIG. 8, the positioning element 12 of the minimally invasive screw device 1 is disposed on the vertebra 85a in a manner that allows rotatation relative to the screw element 11, and the muscle tissues 84 on the surgical path P2 is supported by the expanding portion 122 of the positioning element 12, and the epidermis layer 81, the dermis layer 82 and the subcutaneous tissues 83 are hold by the part of the flexible extension wing 13 exposed from the first section 13a of the positioning element 12.

Similarly, the positioning element 12' of the minimally invasive screw device 1' is disposed on the vertebra 85b in a manner that allows rotation relative to the screw element 11' The expanding portion 122' of the positioning element 12' supports the muscle tissue 84 along the surgical path P2, while the epidermis layer 81, the dermis layer 82 and the subcutaneous tissues 83 are hold by the part of the flexible extension wings 13' exposed from the first section 13a' of the positioning element 12'. Since the elasticity of the epidermis layer 81, the dermis layer 82 and the subcutaneous tissues 83 is relatively smaller than the elasticity of the muscle tissues 84, the epidermis layer 81, the dermis layer 82 and the subcutaneous tissues 83 only require relatively small force to be supported to form the surgical path P2.

Therefore, the flexible extension wings 13, 13' can be formed of a metal wire structure, the dimensions or width of which is significantly smaller than the supporting wall structure of the expanding portions 122, 122'. The size of the incision 80 can be subsequently adjusted by pulling the second sections 13b, 13b' of the flexible extension wings 13, 13' and attaching the second sections 13b, 13b' to the epidermis layer 81 (since the fixing parts 13c', 13c' are oriented away from the epidermis layer 81), and when the tension of the flexible extension wings 13, 13' is released, the opening 80 returns to the original incision size. Therefore, the size of the opening 80 does not need to correspond to the projected areas of the vertebra 85a and the vertebra 85b, and there is no need to open openings 80 of corresponding sizes according to the number of minimally invasive screw devices to obtain a minimized incision 80.

In view of the above, the present disclosure provides a minimally invasive surgical screw device, which includes: a screw element; a positioning element having a limiting portion for receiving the screw element and an expanding portion coupled to the limiting portion; and a flexible extension wing having a first section and a second section, wherein the first section is coupled to the positioning element, and a first bending point is provided between the second section and the first section. Since the size of the flexible extension wing disclosed in the present disclosure is designed to be relatively smaller than that of the positioning element, during the operation of screw device fixation, the muscle tissues can be supported by the positioning element, and the epidermis layer, the dermis layer and the subcutaneous tissues can be supported by the flexible extension wing; therefore, compared with the extension part of conventional vertebral arch screw, the use of the minimally invasive surgical screw device of the present disclosure can perform the installation of the vertebral arch screw while minimizing the wound area on the surgical site, and reduce the space occupied by the surgical path from the skin to the subcutaneous tissues.

Further, since the flexible extension wing of the minimally invasive surgical screw device of the present disclosure is designed using a metal wire structure, which occupies less space in the surgical path, the device, compared with the cylindrical extension parts of the conventional vertebral arch screw, the minimally invasive surgical screw device of the present disclosure can provide a better surgical view and increase the convenience of setting the connecting rod onto the positioning element.

The above embodiments are merely illustrative the technical principles of, features and functions of the present disclosure and should not be construed as to limit the scope of the present disclosure. A person skilled in the art may make modifications and variations to the above embodiments without departing from the spirit and scope of the present disclosure. Modifications and changes are made to the above embodiments. However, any equivalent modifications and changes accomplished by applying the teachings of this disclosure should still be covered by the scope of the following claims. The scope of legal protection of the present disclosure shall be defined by the following claims.

## Claims

1. A minimally invasive surgical screw device, comprising:
a screw element;
a positioning element having a first side and a second side opposite the first side, wherein the positioning element comprises a limiting portion disposed on the first side for receiving the screw element, and an expanding portion connected to the limiting portion and extending toward the second side; and
a flexible extension wing having two opposing first sections respectively coupled to opposite positions of the positioning element, wherein the two first sections extend in a direction parallel to a longitudinal direction of the positioning element.

2. The minimally invasive surgical screw device of claim 1, wherein the limiting portion comprises a recessed opening through which the screw element is inserted into the limiting portion in a relatively rotatable manner.

3. The minimally invasive surgical screw device of claim 1 or 2, wherein the expanding portion comprises two first guide portions, and the two first sections of the flexible extension wing are extended through the two first guide portions to be secured to the positioning element.

4. The minimally invasive surgical screw device of claim 3, wherein each of the two first guide portions is disposed on a peripheral surface of the expanding portion.

5. The minimally invasive surgical screw device of claim 3 or 4, wherein the limiting portion comprises two second guide portions, respectively corresponding to each of the first guide portions, and the two first sections extend through the two first guide portions and further extended through the two second guide portions.

6. The minimally invasive surgical screw device of any one of the preceding claims, wherein the flexible extension wing comprises two second sections respectively connected to each of the first sections, each second section and corresponding first section being connected via a first bending point is bent such that the first section and the second section of the flexible extension wing form an angle of 60 to 120 degrees.

7. The minimally invasive surgical screw device of claim 6, wherein the second section of the flexible extension wing includes at least a second bending point.

8. The minimally invasive surgical screw device of claim 2, wherein the limiting portion comprises a first side wall and a second side wall opposite the first side wall, wherein an accommodating space for receiving the screw element is defined by the first side wall, the second side wall and the recessed opening, and inner surfaces of the first side wall and the second side wall are formed with positioning threads.

9. The minimally invasive surgical screw device of any one of the preceding claims, wherein the expanding portion comprises a first support wall and a second support wall opposite to the first support wall, wherein the first support wall and the second support wall are respectively connected to the limiting portion and extend to the second side of the positioning element so as to define a guide channel extending to the limiting portion.

10. The minimally invasive surgical screw device of claim 9, wherein a width of the first support wall and the second support wall adjacent to the second side of the positioning element is less than a width of the first support wall and the second support wall adjacent to the first side of the positioning element.

11. The minimally invasive surgical screw device of claim 10, wherein a width of the flexible extension wing is less than or equal to a width of the first support wall and the second support wall located on the second side of the positioning element.

12. The minimally invasive surgical screw device of any one of the preceding claims, wherein the flexible extension wing is formed from biocompatible flexible materials comprising metal wires, alloy wires, shape-memory alloys, polymeric fibers, or composites thereof.
